# EUROPEAN PATENT SPECIFICATION

(11) **EP 4 358 836 B1**
(45) Date of publication and mention of the grant of the patent: **11.11.2026**
(21) Application number: 22764905.0
(22) Date of filing: 22.06.2022
(51) Int. Cl.: A61B 5/06

(54) **MEDICAL DEVICE MAGNETIZER SYSTEM WITH INDICATORS**
MAGNETISIERSYSTEM FÜR MEDIZINISCHE VORRICHTUNG MIT INDIKATOREN
SYSTÈME DE MAGNÉTISEUR DE DISPOSITIF MÉDICAL AVEC INDICATEURS

(30) Priority: 22.06.2021 US 202163213539 P
(43) Date of publication of application: 01.05.2024
(73) Proprietor: Bard Access Systems, Inc., Salt Lake City, UT 84116 (US)
(72) Inventor: SOWARDS, Steffan, Salt Lake City, UT 84124 (US); MISENER, Anthony, K., Bountiful, UT 84010 (US); MCLAUGHLIN, William, Robert, Bountiful, UT 84010 (US)
(74) Representative: Hoffmann Eitle
(86) International application number: PCT/US2022/034584
(87) International publication number: WO 2022/271864

(56) References cited:
- US-A- 5 055 813
- US-A1- 2017 325 713
- US-A1- 2018 310 955
- US-A1- 2020 360 662

## Description

### PRIORITY

This application claims the benefit of priority to U.S. Provisional Application No. 63/213,539, filed June 22, 2021.

### BACKGROUND

A ferrous medical device can be magnetized for the purpose of tracking the medical device in three-dimensional space. The ferrous medical device can be brought into a magnetic field within a magnetizer, imprinting a magnetic signature onto the medical device that is able to be tracked in three-dimensional space. However, it can be difficult to determine if the medical device has been magnetized. It would be beneficial to a user to be able to determine if a medical device has been magnetized. Disclosed herein are a system and method of use that address the foregoing. US 2018/310955 A1 discloses a magnetizing system for selectively magnetizing a needle of a medical device, the system including an orientation key system to ensure the needle is positioned correctly in the magnetizing system. US 2020/360662 A1 discloses a device for accommodating and magnetizing a tissue-penetrating medical device of various lengths. US 2017/325713 A1 discloses a cover for magnetizing a shaft of a tissue-penetrating device. US 5055813 A discloses a magnetization/demagnetization device for magnetizing screwdrivers or the like.

### SUMMARY

Disclosed herein is a magnetizer system for use with a medical device including ferrous elements, the system including a magnetizer having a magnetizer body defining a cavity, the magnetizer body having a body opening in communication with the cavity, the magnetizer body including one or more magnets configured to generate a magnetic field configured to imprint a magnetic signature on ferrous elements within the cavity and one or more mechanisms configured to detect the presence of the medical device within the cavity.

The one or more mechanisms include an infrared detection system having one or more infrared emitters configured to generate an infrared beam across the cavity and one or more infrared sensors configured to detect the infrared beam, the infrared emitters and infrared sensors in communication with a console.

In some embodiments, the one or more mechanisms include a mechanical switch configured to transition between a non-magnetized configuration and a magnetized configuration.

In some embodiments, the magnetizer includes a RFID reader coupled to the magnetizer body or contained within the cavity, the RFID reader in communication with the console.

In some embodiments, the medical device includes a RFID tag configured to be detected by the RFID reader.

In some embodiments, the magnetic signature includes a monopole magnetic signature.

In some embodiments, the mechanical switch includes a hinge coupled to each of the mechanical switch and a lever arm, the lever arm protruding into the cavity and configured to be contacted by the medical device to transition the mechanical switch from the non-magnetized configuration to the magnetized configuration.

In some embodiments, the mechanical switch resides within a recess within the magnetizer body.

In some embodiments, the non-magnetized configuration includes the mechanical switch is perpendicular to the lever arm and residing fully within the recess.

In some embodiments, the magnetized configuration includes the mechanical switch moved away from the recess and the magnetizer body.

In some embodiments, the medical device including ferrous elements is selected from the group consisting of a needle, a catheter, and a combination.

In some embodiments, the magnetizer includes one or more sensors in communication with the console, the one or more sensors configured to detect and track the medical device using inductive sensing or optical sensing.

In some embodiments, the console includes one or more processors, an energy source, non-transitory computer-readable medium, and a plurality of logic modules.

In some embodiments, the one or more magnets include one or more electromagnets in communication with the energy source.

In some embodiments, the console is in wireless communication with one or more computing devices or an electronic medical record system.

In some embodiments, the plurality of logic modules, when executed by the processor, are configured to perform operations including activating the one or more infrared sensors and the one or more infrared emitters, detecting the RFID tag of the medical device, detecting the one or more infrared beams from the one or more infrared emitters, activating the one or more sensors, and detecting the medical device using the one or more sensors by inductive sensing or optical sensing.

Also disclosed herein is a method of imprinting a magnetic signature onto a medical device having ferrous elements using a magnetizer system including detecting the medical device with a magnetizer system having one or more mechanisms configured to detect the presence of the medical device, wherein the one or more mechanisms include an infrared detection system having one or more infrared emitters configured to generate an infrared beam across the cavity and one or more infrared sensors configured to detect the infrared beam, the infrared emitters and infrared sensors in communication with a console, placing the medical device within a cavity of the magnetizer, confirming the medical device is within the cavity, and imprinting the magnetic signature onto the medical device.

In some embodiments, the magnetizer system includes a magnetizer having a magnetizer body defining the cavity, the magnetizer body having a body opening in communication with the cavity and one or more magnets configured to imprint the magnetic signature onto the medical device within the cavity; the magnetizer having one or more sensors in communication with a console, the one or more sensors configure to detect and track the medical device using inductive sensing or optical sensing; and a mechanical switch configured to transition between a non-magnetized configuration and a magnetized configuration.

In some embodiments, the magnetizer includes a RFID reader coupled to the magnetizer body or contained within the cavity, the RFID reader in communication with the console and configured to detect a RFID tag coupled to the medical device.

In some embodiments, detecting the medical device includes using the RFID reader to detect the RFID tag of the medical device.

In some embodiments, placing the medical device within the cavity of the magnetizer include slidably inserting the medical device within the cavity.

In some embodiments, the infrared detection system confirms the medical device is within the cavity when the medical device breaks the infrared beam across the cavity.

In some embodiments, confirming the medical device is within the cavity includes confirming by a mechanical switch coupled to the magnetizer transitioned from a non-magnetized configuration to a magnetized configuration.

In some embodiments, the mechanical switch resides within a recess of the magnetizer body, having a hinge coupled to the mechanical switch and a lever arm, the lever arm protruding into the cavity and configured to be contacted by the medical device.

In some embodiments, imprinting the magnetic signature onto the medical device includes imprinting the magnetic signature onto the medical device as the medical device is removed from the cavity.

In some embodiments, the magnetic signature includes a monopole magnetic signature.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a cross sectional view of a magnetizer system including a magnetizer and a medical device, in accordance with some embodiments.
FIG. 2A illustrates a cross sectional view of the magnetizer having an infrared detection system, in accordance with some embodiments.
FIGS. 2B-2C illustrates a cross sectional view of the exemplary method of the infrared detection system detecting the medical device, in accordance with some embodiments.
FIGS. 3A-3B illustrates a cross sectional view of a mechanical switch transitioning between a non-magnetized configuration and a magnetized configuration, in accordance with some embodiments.
FIG. 4 illustrates a cross sectional view of the magnetizer including a plurality of magnets and a RFID reader, in accordance with some embodiments.
FIG. 5 illustrates a block diagram of some components of the magnetizer including a console, in accordance with some embodiments.
FIGS. 6A-6C illustrates a cross sectional view of an exemplary method of providing a magnetic signature to the medical device using the magnetizer, in accordance with some embodiments.
FIG. 7 illustrates a flow chart of the exemplary method of providing a magnetic signature to a medical device using the magnetizer, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

The term "logic" may be representative of hardware, firmware or software that is configured to perform one or more functions. As hardware, the term logic may refer to or include circuitry having data processing and/or storage functionality. Examples of such circuitry may include, but are not limited or restricted to a hardware processor (e.g., microprocessor, one or more processor cores, a digital signal processor, a programmable gate array, a microcontroller, an application specific integrated circuit "ASIC", etc.), a semiconductor memory, or combinatorial elements.

Additionally, or in the alternative, the term logic may refer to or include software such as one or more processes, one or more instances, Application Programming Interface(s) (API), subroutine(s), function(s), applet(s), servlet(s), routine(s), source code, object code, shared library/dynamic link library (dll), or even one or more instructions. This software may be stored in any type of a suitable non-transitory storage medium, or transitory storage medium (e.g., electrical, optical, acoustical, or other form of propagated signals such as carrier waves, infrared signals, or digital signals). Examples of a non-transitory storage medium may include, but are not limited or restricted to a programmable circuit; non-persistent storage such as volatile memory (e.g., any type of random access memory "RAM"); or persistent storage such as non-volatile memory (e.g., read-only memory "ROM", power-backed RAM, flash memory, phase-change memory, etc.), a solid-state drive, hard disk drive, an optical disc drive, or a portable memory device. As firmware, the logic may be stored in persistent storage.

Unless defined otherwise, all technical and scientific terms used herein have the same meaning as commonly understood by those of ordinary skill in the art.

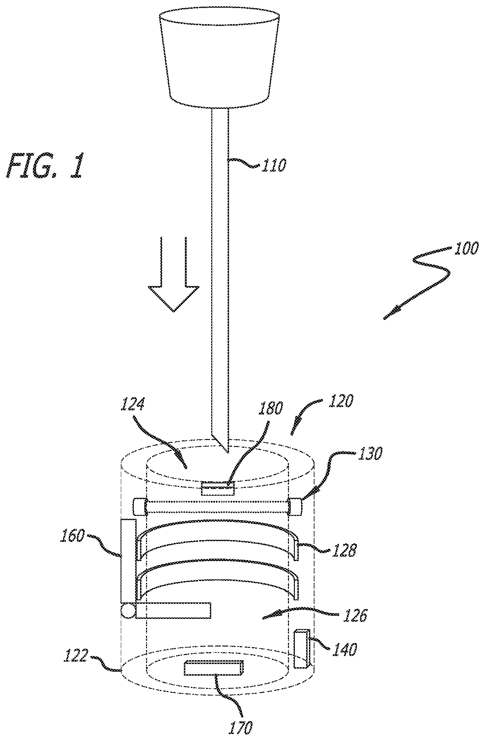

FIG. 1 illustrates a cross sectional view of a magnetizer system 100 including a magnetizer 120 and a medical device 110, in accordance with some embodiments. The magnetizer system 100 includes the magnetizer 120 configured to imprint a magnetic signature upon the medical device 110 having ferrous elements therein. The magnetic signature may allow tracking of the location and orientation of the medical device 110 in subsequent use. In some embodiments, the medical device 110 may include a needle, a vascular access device including a catheter, a peripherally inserted central catheter, or the like. The magnetizer 120 includes a magnetizer body 122 defining a cavity 126 therein. The magnetizer 120 includes a body opening 124 in communication with the cavity 126, the body opening 124 allowing the medical device 110 to be slidably received therethrough. In some embodiments, the magnetizer body 122 may be shaped in a cylindrical prism, a rectangular prism or the like. The magnetizer 120 includes one or more mechanisms for detecting the presence of the medical device 110 within the cavity 126. Such detecting mechanisms may also indicate to a user that the medical device 110 has been inserted, or magnetized, or that the magnetizer 120 has been activated. The detecting mechanisms include an infrared detection system 130. In some embodiments, the detecting mechanisms may include one or more of a mechanical switch 160, one or more magnets 128 and/or a RFID reader 170. In some embodiments, the magnetizer 120 may include one or more sensors 180 configured to detect and track the medical device 110. The one or more sensors 180 may be coupled to the magnetizer 120 and/or may be located within the cavity 126. In some embodiments, the one or more sensors 180 may be wired to a console 140 or may be in wireless communication with the console 140. In some embodiments, the one or more sensors 180 may include sensors configured for inductive sensing or optical sensing of the medical device 110.

The magnetic signature may be consistent with differentiating information/data regarding the medical device 110 such that a first subset of a plurality of the medical devices 110 includes a magnetic signature that is different from the magnetic signature of a second subset of the plurality of the medical devices 110. In some embodiments, the differentiating information may include model information for the medical device 110, such as a module name or model number, for example. In some embodiments, the differentiating information may include dimensional information of the medical device 110, such as a length or diameter, for example. In some embodiments, the differentiating information may include manufacturing information for the medical device 110, such as a manufacturing date or lot number, for example. In some embodiments, the differentiating information may include unique information pertaining to the medical device 110, such as a serial number, for example. As such, in some embodiments, the magnetic signature for any one medical device 110 may be unique with respect to (i.e., different from) the magnetic signature for every other medical device 110.

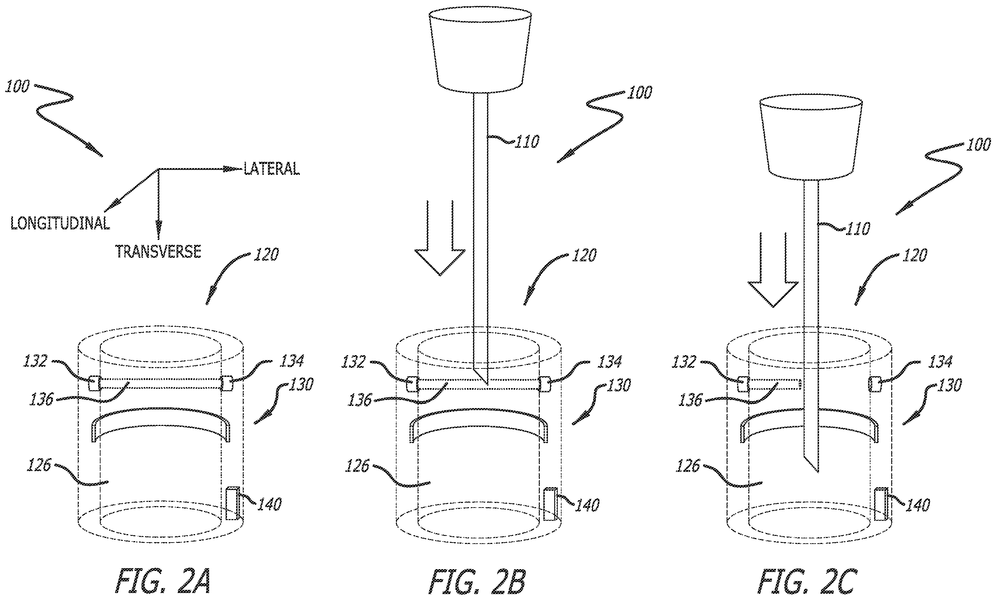

FIG. 2A illustrates a cross sectional view of the magnetizer 120 having the infrared detection system 130, in accordance with some embodiments. The infrared detection system 130 may include one or more infrared ("IR") emitters 132 and one or more corresponding IR sensors 134 in communication with the console 140. The one or more IR emitters 132 and one or more IR sensors 134 may be wired to the console 140 or in wireless communication with the console 140. In some embodiments, the IR emitters 132 and the IR sensors 134 may also include near infrared ("NIR") emitters and sensors. Each IR emitter 132 may be configured to emit an IR beam 136 spanning the cavity 126 so that the IR beam 136 is detected by the corresponding IR sensor 134. Each IR emitter 132 may be arranged laterally in line with each corresponding IR sensor 134. In some embodiments, the IR emitters 132 and corresponding IR sensors 134 may be located adjacent the body opening 124. In some embodiments, the IR emitter 132 may be configured to continuously emit the IR beam 136 and the IR sensor 134 may be configured to continuously detect the IR beam 136. In some embodiments, the IR emitter 132 may be configured to emit the IR beam 136 in accordance with a user defined interval cycle (e.g., every 5 milliseconds, every second or the like) and the IR sensor 134 may be configured to detect the IR beam 136 in accordance with the user defined interval cycle.

FIGS. 2B-2C illustrates a cross sectional view depicting an exemplary method of the magnetizer 120 detecting the medical device 110 within the cavity 126 using the IR detection system 130, in accordance with some embodiments. As illustrated in FIG. 2B, the medical device 110 may be inserted into the magnetizer 120 to be magnetized. As illustrated in FIG. 2C, as the medical device 110 is inserted into the cavity, the medical device 110 may interrupt the IR beam 136. The IR sensor 134 may be configured to (i) detect the interruption of the IR beam 136 and (ii) transmit the detected interruption to the console 140. The console 140 may generate an alert and wirelessly transmit (e.g., wirelessly transmit) the alert to a computing device or an electronic medical record (EMR) system indicating the medical device 110 has been magnetized. When the medical device 110 is fully removed from the cavity 126, the IR beam 136 will be restored and detected by the IR sensor 134

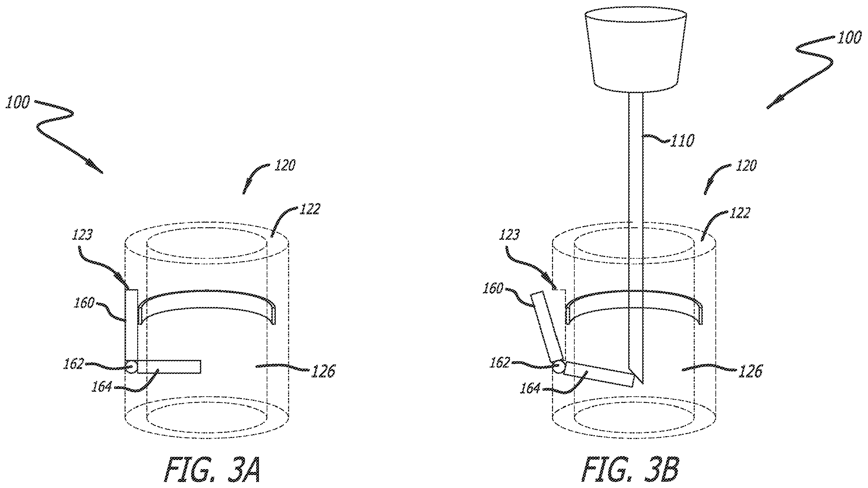

FIGS. 3A-3B illustrates a cross sectional view of the magnetizer 120 including the mechanical switch 160, in accordance with some embodiments. In some embodiments, the magnetizer 120 includes the mechanical switch ("switch") 160 coupled to the magnetizer body 122, the switch 160 configured to indicate to a user when the medical device 110 is within the cavity 126. The switch 160 may reside within a recess 123 in the magnetizer body 122, the switch 160 may be configured to transition from a non-magnetized configuration (i.e., a configuration or position of the switch 160 consistent with the medical device 110 in a non-magnetized state) to a magnetized configuration (i.e., a configuration or position of the switch 160 consistent with the medical device 110 in a magnetized state). The switch 160 may be coupled to a hinge 162, where the hinge 162 may reside at the bottom of the recess 123. The hinge 162 may be configured to allow the switch 160 to transition between the non-magnetized configuration and the magnetized configuration. The hinge 162 may be further coupled to a lever arm 164. The lever arm 164 may be configured to (i) protrude into the cavity 126 and (ii) contact the medical device 110 as the medical device 110 is inserted into the cavity 126. The downward motion of the medical device 110 contacting the lever arm 164 may transition the switch 160 from the non-magnetized configuration to the magnetized configuration. In some embodiments, in the non-magnetized configuration, the switch 160 may reside fully within the recess 123, perpendicular to the lever arm 164 as illustrated in FIG. 3A. In some embodiments, in the magnetized configuration, the switch 160 may extend away from the recess 123 and the magnetizer body 122, as illustrated in FIG. 3B. The lever arm 164 may be configured to deflect or move downward under force. As the medical device 110 is displaced downward within the cavity 126, the medical device 110 contacts the lever arm 164 so that the lever arm 164 deflects downward. As the lever arm 164 deflects downward, the mechanical switch 160 may displace away from the magnetizer 120, signaling that the medical device 110 is (i) within the cavity 126 and (ii) has been magnetized. Once the switch 160 has been transitioned to the magnetized configuration, the switch 160 may be reset to the non-magnetized configuration. Other mechanisms of transitioning the mechanical switch 160 from the non-magnetized configuration to the magnetized configuration are considered.

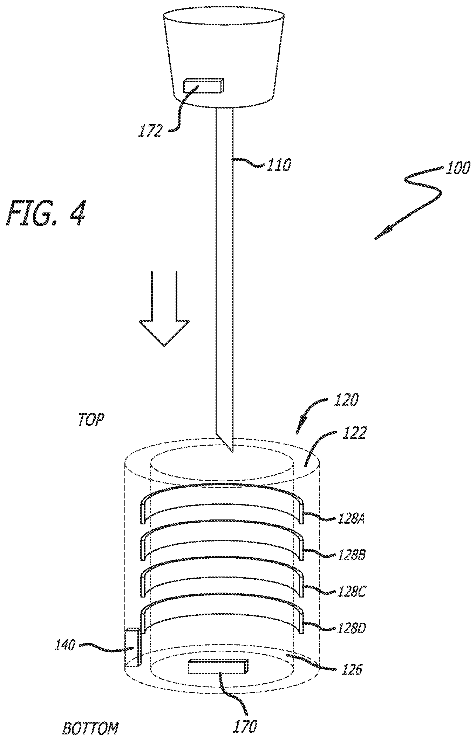

FIG. 4 illustrates a cross sectional view of the magnetizer 120 including the one or more magnets 128A-128D arranged around the cavity 126 and the RFID reader 170, in accordance with some embodiments. In some embodiments, the one or more magnets 128A-128D may be configured to generate the magnetic field within the cavity 126. In some embodiments, the one or more magnets 128A-128D may be configured to generate a monopole magnetic field configured to magnetize ferrous elements disposed within the cavity 126 as the ferrous elements are withdrawn from the magnetic field, thereby imprinting the magnetic signature to the ferrous elements. In some embodiments, the magnetic signature may include a monopole magnetic signature. In some embodiments, the one or more magnets 128A-128D may include one or more electromagnets operatively coupled with an energy source. In some embodiments, the one or more magnets 128A-128D may include one or more magnets that wrap annularly around the cavity 126. In some embodiments, the magnetizer body 122 may include magnetic shielding materials (not shown) configured to contain the magnetic field generated by the one or more magnets 128A-128D within the cavity 126.

In some embodiments, the RFID reader 170 may be in communication with the console 140 or other electronic devices. The RFID reader 170 may be configured to detect one or more RFID tags brought near the magnetizer 120. In some embodiments, the RFID reader 170 may be coupled to the magnetizer 120 and/or may be located within the cavity 126. For example, the RFID reader 170 may be located at a bottom of the cavity 126. In some embodiments, the medical device 110 may include a RFID tag 172 containing information about the medical device 110. Details of an exemplary RFID tag and RFID reader system can be found in US 2018/0310955, published November 1, 2018. The RFID reader 170 may communicate information contained within a detected RFID tag 172 to the console 140, and in some embodiments, the console 140 may communicate the information to additional electronic devices.

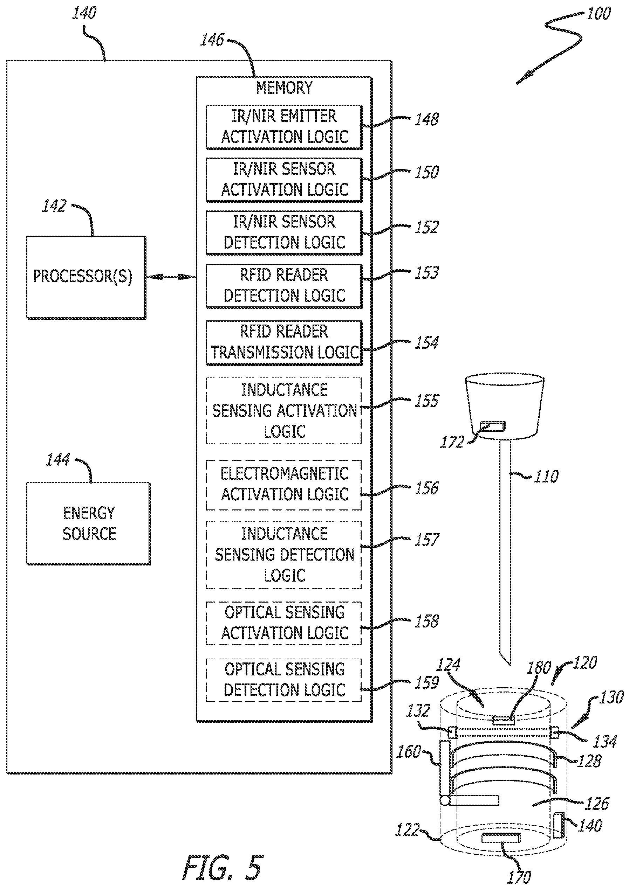

FIG. 5 illustrates a block diagram of some components of the magnetizer 120 including the console 140, in accordance with some embodiments. In some embodiments, the console 140 may include one or more processors 142, an energy source 144, non-transitory computer-readable storage medium ("memory") 146 and a plurality of logic modules. In some embodiments, the energy source 144 may be configured to provide power to the console 140, the IR detection system 130 including the IR/NIR emitters 132, the IR/NIR sensors 134, the one or more sensors 180, and the one or more electromagnets 128. In some embodiments, the energy source 144 may include a rechargeable battery. In some embodiments, the rechargeable battery may be recharged through induction.

In some embodiments, the plurality of logic modules may be configured to include one or more of: an IR/NIR emitter activation logic 148, an IR/NIR sensor activation logic 150, an IR/NIR sensor detection logic 152, an RFID reader detection logic 153, an RFID reader transmission logic 154, and an electromagnet energy source activation logic 156. In some embodiments, the IR/NIR emitter activation logic 148 may be configured to activate the one or more IR/NIR emitters 132 to generate the IR beam 136. In some embodiments, the IR/NIR sensor activation logic 150 may be configured to activate the one or more IR/NIR sensors 134 to detect the IR beam 136. In some embodiments, the IR/NIR sensor detection logic 152 may be configured to detect the IR beam 136 from the one or more emitters 132. In some embodiments, the RFID reader detection logic 153 may be configured to detect the one or more RFID tags brought into communication with the RFID reader 170. In some embodiments, the RFID reader detection logic 153 may be configured to continuously detect the one or more RFID tags, or the RFID reader detection logic 153 may be configured to detect the one or more RFID tags in accordance with a user defined time interval. In some embodiments, the RFID reader detection logic 153 may activate the IR/NIR emitter activation logic 148 and the IR/NIR sensor activation logic 150.

By way of example, a medical device 110 having the RFID tag 172 may be brought into the vicinity of the RFID reader 170. The RFID reader 170 may detect the RFID tag 172 and activate the IR detection system 130. In some embodiments, the RFID reader transmission logic 154 may be configured to wireless transmit the detected RFID tag information to other electronic devices including computing devices, ultrasound probes, the EMR system, or the like. In some embodiments, the RFID reader transmission logic 154 may be configured to transmit an alert, to a computing device, indicating that the medical device 110 has been magnetized. In some embodiments, wherein the one or more magnets 128 are electromagnets, the electromagnetic activation logic 156 may be configured activate the one or more electromagnets 128 to generate the magnetic field within the cavity 126. In some embodiments, where the magnetizer 120 includes the one or more sensors 180 configured to track the medical device 110 using inductive sensing or optical sensing, the plurality of logic modules may include an inductive sensing activation logic 155, an inductive sensing detection logic 157, an optical sensing activation logic 158, and an optical sensing detection logic 159. In some embodiments, the inductive sensing activation logic 155 may be configured to activate the one or more sensors 180 for inductive sensing. In some embodiments, the inductive sensing detection logic 157 may be configured to detect and track the medical device 110 using inductive sensing via the one or more sensors 180. In some embodiments, the optical sensing activation logic 158 may be configured to activate the one or more sensors 180 for optical sensing. In some embodiments, the optical sensing detection logic 159 may be configured to detect and track the medical device 110 using optical sensing via the one or more sensors 180.

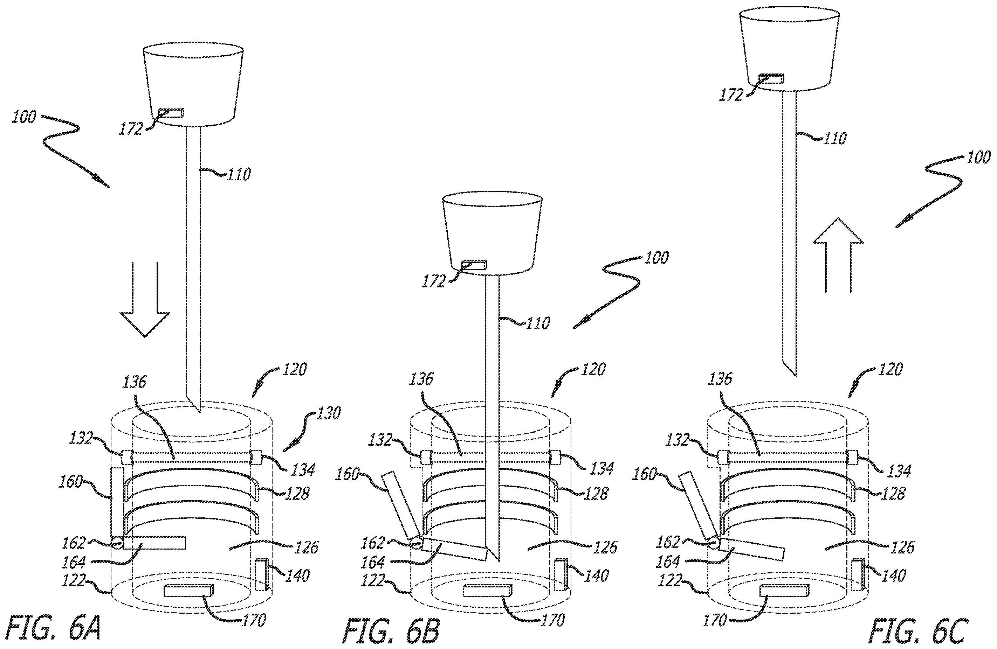

FIGS. 6A-6C illustrate a cross sectional views depicting an exemplary method of providing a magnetic signature to the medical device 110 using the magnetizer 120, in accordance with some embodiments. In some embodiments, the magnetizer 120 includes the one or more magnets 128, the mechanical switch 160, the IR detection system 130, and the RFID reader 170, as described above. The mechanical switch 160 may be in the non-magnetized configuration and the IR/NIR sensors 134 may detect an unbroken IR beam 136 from the IR/NIR emitters 132, as illustrated in FIG. 6A. The medical device 110 having the RFID tag 172 may be inserted into the cavity 126 of the magnetizer 120, breaking the IR beam 136. As illustrated in FIG. 6B, the medical device 110 may be inserted into the cavity 126 wherein the IR/NIR sensors 134 may detect the broken IR beam from the IR/NIR emitters 132, and the medical device may contact the lever arm 164. The RFID reader 170 may detect the RFID tag 172 of the medical device 110 and communicate the information contained in the RFID tag 172 to the console 140. The mechanical switch 160 may transition from the non-magnetized configuration to the magnetized configuration. During removal of the medical device 110 from the cavity 126, the one or more magnets 128 may magnetize the medical device 110 with the magnetic signature. When the medical device 110 is fully removed from the cavity 126, the IR/NIR sensors 134 may detect the restored IR beam 136 from the IR/NIR emitters 132. The mechanical switch 160 may indicate the medical device 110 has been magnetized via the mechanical switch 160 having transitioned to the magnetized configuration. Once the medical device 110 has been fully removed from the cavity 126, the mechanical switch 160 may be reset to the non-magnetized configuration, and the IR beam 136 may be restored so as to be detected by the IR/NIR sensors 134.

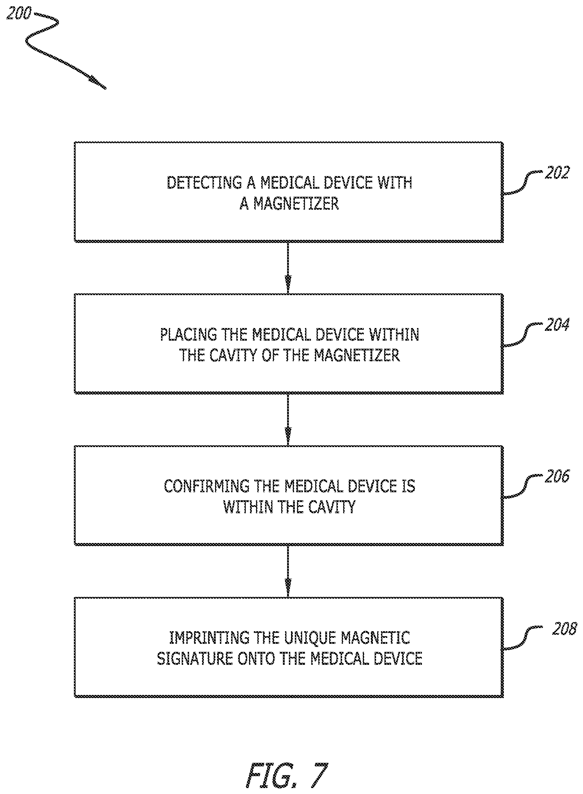

FIG. 7 illustrates a flow chart of an exemplary method 200 of providing a magnetic signature to the medical device 110 using the magnetizer 120, in accordance with some embodiments. In some embodiments, the method 200 includes detecting the medical device 110 with the magnetizer 120 (block 202). In some embodiments, detecting the medical device 110 includes detecting the medical device 110 with the RFID reader 170 coupled to the magnetizer 120. In some embodiments, detecting the medical device 110 with the RFID reader 170 includes detecting the RFID tag 172 coupled to the medical device 110. In some embodiments, detecting the medical device 110 includes using the one or more sensors 180 to detect the medical device 110 via inductive sensing or optical sensing.

The method 200 further includes placing the medical device 110 within the cavity 126 of the magnetizer 120 (block 204). In some embodiments, placing the medical device 110 within the cavity 126 includes slidably inserting, dipping or otherwise displacing the medical device 110 into the cavity 126. The method 200 includes confirming the medical device 110 is within the cavity 126 (block 206). In some embodiments, confirming the medical device 110 is within the cavity 126 includes confirming the medical device 110 is within the cavity via the IR detection system 130. In some embodiments, confirming with the IR detection system 130 includes confirming via the medical device 110 interrupting the IR beam 136. In some embodiments, confirming the medical device 110 is within the cavity 126 includes confirming via the mechanical switch 160 transitioning from the non-magnetized configuration to the magnetized configuration. In some embodiments, the medical device 110 contacting the lever arm 164 transitions the mechanical switch 160 from the non-magnetized configuration to the magnetized configuration.

The method 200 further includes imprinting the magnetic signature onto medical device 110 (block 208). In some embodiments, imprinting the magnetic signature onto the medical device 110 includes the one or more magnets 128 imprinting the magnetic signature onto the medical device 110. In some embodiments, the process of withdrawing the medical device 110 from the cavity 126 imprints the magnetic signature onto the medical device 110.

While some particular embodiments have been disclosed herein, and while the particular embodiments have been disclosed in some detail, it is not the intention for the particular embodiments to limit the scope of the claims. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the appended claims.

## Claims

1. A magnetizer system (100) for use with a medical device (110) including ferrous elements, the system comprising:
a magnetizer (120) having a magnetizer body (122) defining a cavity (126), the magnetizer body having a body opening (124) in communication with the cavity, the magnetizer body including one or more magnets (128) configured to generate a magnetic field configured to imprint a magnetic signature on ferrous elements within the cavity;
one or more mechanisms configured to detect the presence of the medical device within the cavity;
**characterized in that** the one or more mechanisms include an infrared detection system (130) having one or more infrared emitters (132) configured to generate an infrared beam across the cavity and one or more infrared sensors (134) configured to detect the infrared beam, the infrared emitters, and infrared sensors in communication with a console (140).

2. The magnetizer system according to claim 1, wherein the magnetic signature is unique.

3. The magnetizer system according to either claim 1 or claim 2, wherein the one or more mechanisms include a mechanical switch (160) configured to transition between a non-magnetized configuration and a magnetized configuration.

4. The magnetizer system according to any claim of claims 1-3, wherein the magnetic signature includes a monopole magnetic signature and/or the magnetizer includes a RFID reader (170) coupled to the magnetizer body or contained within the cavity, the RFID reader in communication with the console; and optionally wherein the medical device includes a RFID tag (172) configured to be detected by the RFID reader.

5. The magnetizer system according to any claim of claims 3 or 4, wherein the mechanical switch includes a hinge (162) coupled to each of the mechanical switch and a lever arm (164), the lever arm protruding into the cavity and configured to be contacted by the medical device to transition the mechanical switch from the non-magnetized configuration to the magnetized configuration; and optionally
wherein the magnetized configuration includes the mechanical switch moved away from the recess and the magnetizer body; or wherein the mechanical switch resides within a recess within the magnetizer body; and further optionally wherein the non-magnetized configuration includes the mechanical switch is perpendicular to the lever arm and residing fully within the recess.

6. The magnetizer system according to any claim of claims 1-5, wherein the medical device including ferrous elements is selected from the group consisting of a needle, a catheter, and combinations thereof.

7. The magnetizer system according to any claim of claims 2-6, wherein the magnetizer includes one or more sensors in communication with the console, the one or more sensors configured to detect and track the medical device using inductive sensing or optical sensing.

8. The magnetizer system according to any claim of claims 2-7, wherein the console includes one or more processors (142), an energy source (144), non-transitory computer-readable medium (146), and a plurality of logic modules; and optionally wherein the one or more magnets include one or more electromagnets (128) in communication with the energy source, or wherein the console is in wireless communication with one or more computing devices or an electronic medical record system.

9. The magnetizer system according to claim 8, wherein the plurality of logic modules, when executed by the processor, are configured to perform operations including:
activating the one or more infrared sensors and the one or more infrared emitters;
detecting the RFID tag of the medical device;
detecting the one or more infrared beams from the one or more infrared emitters;
activating the one or more sensors; and
detecting the medical device using the one or more sensors by inductive sensing or optical sensing.

10. A method of imprinting a magnetic signature onto a medical device (110) having ferrous elements using a magnetizer system (100), comprising:
detecting the medical device (110) with a magnetizer system (100) having one or more mechanisms configured to detect the presence of the medical device wherein the one or more mechanisms include an infrared detection system (130) having one or more infrared emitters (132) configured to generate an infrared beam across a cavity of the magnetizer and one or more infrared sensors (134) configured to detect the infrared beam, the infrared emitters, and infrared sensors in communication with a console (140);
placing the medical device within the cavity (126) of the magnetizer;
confirming the medical device is within the cavity; and
imprinting the magnetic signature onto the medical device.

11. The method according to claim 10, wherein the magnetic signature is unique and/or
wherein the magnetizer system includes a magnetizer (120) having a magnetizer body (122) defining the cavity (126), the magnetizer body having a body opening (124) in communication with the cavity and one or more magnets configured to imprint the magnetic signature onto the medical device within the cavity;
the magnetizer having one or more sensors (180) in communication with a console (140), the one or more sensors configure to detect and track the medical device using inductive sensing or optical sensing;
and a mechanical switch (160) configured to transition between a non-magnetized configuration and a magnetized configuration; and optionally
wherein the magnetizer includes a RFID reader (170) coupled to the magnetizer body or contained within the cavity, the RFID reader in communication with the console and configured to detect a RFID tag (172) coupled to the medical device; and
further optionally wherein detecting the medical device includes using the RFID reader to detect the RFID tag of the medical device.

12. The method according to any claim of claims 10 or 11, wherein placing the medical device within the cavity of the magnetizer include slidably inserting the medical device within the cavity.

13. The method according to any claim of claims 11 or 12, wherein the infrared detection system confirms the medical device is within the cavity when the medical device breaks the infrared beam across the cavity.

14. The method according to any claim of claims 11-13, wherein confirming the medical device is within the cavity includes confirming by a mechanical switch coupled to the magnetizer transitioned from a non-magnetized configuration to a magnetized configuration; and optionally wherein the mechanical switch resides within a recess of the magnetizer body, having a hinge (162) coupled to the mechanical switch and a lever arm (164), the lever arm protruding into the cavity and configured to be contacted by the medical device.

15. The method according to any claim of claims 10-14, wherein imprinting the magnetic signature onto the medical device includes imprinting the magnetic signature onto the medical device as the medical device is removed from the cavity; and optionally wherein the magnetic signature includes a monopole magnetic signature.

## Patentansprüche

1. Magnetisiersystem (100) zur Verwendung mit einer medizinischen Vorrichtung (110), welche eisenhaltige Elemente einschließt, das System umfassend:
einen Magnetisierer (120), der einen Magnetisiererkörper (122) aufweist, der einen Hohlraum (126) definiert, wobei der Magnetisiererkörper eine Körperöffnung (124) in Kommunikation mit dem Hohlraum aufweist, wobei der Magnetisiererkörper einen oder mehrere Magnete (128) einschließt, die dazu ausgebildet sind, ein Magnetfeld zu erzeugen, das dazu ausgebildet ist, eisenhaltigen Elementen innerhalb des Hohlraums eine magnetische Signatur aufzuprägen;
einen oder mehrere Mechanismen, die dazu ausgebildet sind, das Vorhandensein der medizinischen Vorrichtung innerhalb des Hohlraums zu detektieren;
**dadurch gekennzeichnet, dass**
der eine oder die mehreren Mechanismen ein Infrarotdetektionssystem (130) einschließen, das einen oder mehrere Infrarotemitter (132), die dazu ausgebildet sind, einen Infrarotstrahl über den Hohlraum hinweg zu erzeugen, und einen oder mehrere Infrarotsensoren (134), die dazu ausgebildet sind, den Infrarotstrahl zu detektieren, aufweist, wobei die Infrarotemitter und die Infrarotsensoren in Kommunikation mit einer Konsole (140) stehen.

2. Magnetisiersystem nach Anspruch 1, wobei die magnetische Signatur eindeutig ist.

3. Magnetisiersystem nach Anspruch 1 oder Anspruch 2, wobei der eine oder die mehreren Mechanismen einen mechanischen Schalter (160) einschließen, der dazu ausgebildet ist, zwischen einer nichtmagnetisierten Konfiguration und einer magnetisierten Konfiguration überzugehen.

4. Magnetisiersystem nach einem der Ansprüche 1-3, wobei die magnetische Signatur eine monopolare magnetische Signatur einschließt und/oder der Magnetisierer einen RFID-Leser (170) einschließt, der an den Magnetisiererkörper gekoppelt ist oder innerhalb des Hohlraums enthalten ist, wobei der RFID-Leser in Kommunikation mit der Konsole steht; und wobei wahlweise die medizinische Vorrichtung ein RFID-Etikett (172) einschließt, das dazu ausgebildet ist, durch den RFID-Leser detektiert zu werden.

5. Magnetisiersystem nach einem der Ansprüche 3 oder 4, wobei der mechanische Schalter ein Gelenk (162) einschließt, das an jedes von dem mechanischen Schalter und einem Hebelarm (164) gekoppelt ist, wobei der Hebelarm in den Hohlraum hervorsteht und dazu ausgebildet ist, durch die medizinische Vorrichtung kontaktiert zu werden, um den mechanischen Schalter aus der nichtmagnetisierten Konfiguration in die magnetisierte Konfiguration zu überführen; und
wobei wahlweise die magnetisierte Konfiguration einschließt, dass der mechanische Schalter von der Aussparung und dem Magnetisiererkörper wegbewegt ist; oder wobei sich der mechanische Schalter innerhalb einer Aussparung innerhalb des Magnetisiererkörpers befindet; und wobei die nichtmagnetisierte Konfiguration weiter wahlweise einschließt, dass der mechanische Schalter senkrecht zu dem Hebelarm ist und sich vollständig innerhalb der Aussparung befindet.

6. Magnetisiersystem nach einem der Ansprüche 1-5, wobei die medizinische Vorrichtung, die eisenhaltige Elemente einschließt, aus der Gruppe bestehend aus einer Nadel, einem Katheter und Kombinationen davon ausgewählt ist.

7. Magnetisiersystem nach einem der Ansprüche 2-6, wobei der Magnetisierer einen oder mehrere Sensoren in Kommunikation mit der Konsole einschließt, wobei der eine oder die mehreren Sensoren dazu ausgebildet sind, die medizinische Vorrichtung unter Verwendung von induktiver Erfassung oder optischer Erfassung zu detektieren und zu verfolgen.

8. Magnetisiersystem nach einem der Ansprüche 2-7, wobei die Konsole einen oder mehrere Prozessoren (142), eine Energiequelle (144), ein nichttransitorisches computerlesbares Medium (146) und eine Vielzahl von Logikmodulen einschließt; und wobei wahlweise der eine oder die mehreren Magnete einen oder mehrere Elektromagnete (128) in Kommunikation mit der Energiequelle einschließen, oder wobei die Konsole in Drahtloskommunikation mit einer oder mehreren Rechenvorrichtungen oder einem elektronischen Patientenaktensystem steht.

9. Magnetisiersystem nach Anspruch 8, wobei die Vielzahl von Logikmodulen dazu ausgebildet ist, bei Ausführung durch den Prozessor Vorgänge durchzuführen, die Folgendes einschließen:
Aktivieren des einen oder der mehreren Infrarotsensoren und des einen oder der mehreren Infrarotemitter;
Detektieren des RFID-Etiketts der medizinischen Vorrichtung;
Detektieren des einen oder der mehreren Infrarotstrahlen von dem einen oder den mehreren Infrarotemittern; Aktivieren des einen oder der mehreren Sensoren; und
Detektieren der medizinischen Vorrichtung unter Verwendung des einen oder der mehreren Sensoren durch induktive Erfassung oder optische Erfassung.

10. Verfahren zum Aufprägen einer magnetischen Signatur auf eine medizinische Vorrichtung (110), die eisenhaltige Elemente aufweist, unter Verwendung eines Magnetisiersystems (100), umfassend:
Detektieren der medizinischen Vorrichtung (110) mit einem Magnetisiersystem (100), das einen oder mehrere Mechanismen aufweist, die dazu ausgebildet sind, das Vorhandensein der medizinischen Vorrichtung zu detektieren, wobei der eine oder die mehreren Mechanismen ein Infrarotdetektionssystem (130) einschließen, das einen oder mehrere Infrarotemitter (132), die dazu ausgebildet sind, einen Infrarotstrahl über den Hohlraum des Magnetisierers hinweg zu erzeugen, und einen oder mehrere Infrarotsensoren (134), die dazu ausgebildet sind, den Infrarotstrahl zu detektieren, aufweist, wobei die Infrarotemitter und die Infrarotsensoren in Kommunikation mit einer Konsole (140) stehen;
Platzieren der medizinischen Vorrichtung innerhalb des Hohlraums (126) des Magnetisierers;
Bestätigen, dass sich die medizinische Vorrichtung innerhalb des Hohlraums befindet; und
Aufprägen der magnetischen Signatur auf die medizinische Vorrichtung.

11. Verfahren nach Anspruch 10, wobei die magnetische Signatur eindeutig ist und/oder
wobei das Magnetisierungssystem einen Magnetisierer (120) einschließt, der einen Magnetisiererkörper (122) aufweist, der den Hohlraum (126) definiert, wobei der Magnetisiererkörper eine Körperöffnung (124) in Kommunikation mit dem Hohlraum und einen oder mehrere Magnete, die dazu ausgebildet sind, die magnetische Signatur auf die medizinische Vorrichtung innerhalb des Hohlraums aufzuprägen, aufweist;
wobei der Magnetisierer einen oder mehrere Sensoren (180) in Kommunikation mit einer Konsole (140) aufweist, wobei der eine oder die mehreren Sensoren dazu ausgebildet sind, die medizinische Vorrichtung unter Verwendung von induktiver Erfassung oder optischer Erfassung zu detektieren und zu verfolgen;
und einen mechanischen Schalter (160), der dazu ausgebildet ist, zwischen einer nichtmagnetisierten Konfiguration und einer magnetisierten Konfiguration überzugehen; und
wobei wahlweise der Magnetisierer einen RFID-Leser (170) einschließt, der an den Magnetisiererkörper gekoppelt ist oder innerhalb des Hohlraums enthalten ist, wobei der RFID-Leser in Kommunikation mit der Konsole steht und dazu ausgebildet ist, ein RFID-Etikett (172) zu detektieren, das an die medizinische Vorrichtung gekoppelt ist; und
wobei weiter wahlweise das Detektieren der medizinischen Vorrichtung Verwenden des RFID-Lesers einschließt, um das RFID-Etikett der medizinischen Vorrichtung zu detektieren.

12. Verfahren nach einem der Ansprüche 10 oder 11, wobei das Platzieren der medizinischen Vorrichtung in den Hohlraum des Magnetisierers verschiebliches Einsetzen der medizinischen Vorrichtung in den Hohlraum einschließt.

13. Verfahren nach einem der Ansprüche 11 oder 12, wobei das Infrarotdetektionssystem bestätigt, dass sich die medizinische Vorrichtung innerhalb des Hohlraums befindet, wenn die medizinische Vorrichtung den Infrarotstrahl über den Hohlraum hinweg unterbricht.

14. Verfahren nach einem der Ansprüche 11-13, wobei das Bestätigen, dass sich die medizinische Vorrichtung innerhalb des Hohlraums befindet, Bestätigen dadurch, dass ein mechanischer Schalter, der an den Magnetisierer gekoppelt ist, aus einer nichtmagnetisierten Konfiguration in eine magnetisierte Konfiguration übergegangen ist, einschließt; und wobei wahlweise der mechanische Schalter sich innerhalb einer Aussparung des Magnetisiererkörpers befindet, aufweisend ein Gelenk (162), das an den mechanischen Schalter und einen Hebelarm (164) gekoppelt ist, wobei der Hebelarm in den Hohlraum hervorsteht und dazu ausgebildet ist, durch die medizinische Vorrichtung kontaktiert zu werden.

15. Verfahren nach einem der Ansprüche 10-14, wobei das Aufprägen der magnetischen Signatur auf die medizinische Vorrichtung Aufprägen der magnetischen Signatur auf die medizinische Vorrichtung, während die medizinische Vorrichtung aus dem Hohlraum entfernt wird, einschließt; und wobei wahlweise die magnetische Signatur eine monopolare magnetische Signatur einschließt.

## Revendications

1. Système de magnétiseur (100) destiné à être utilisé avec un dispositif médical (110) incluant des éléments ferreux, le système comprenant :
un magnétiseur (120) présentant un corps de magnétiseur (122) définissant une cavité (126), le corps de magnétiseur présentant une ouverture de corps (124) en communication avec la cavité, le corps de magnétiseur incluant un ou plusieurs aimants (128) configurés pour générer un champ magnétique configuré pour imprimer une signature magnétique sur des éléments ferreux à l'intérieur de la cavité ;
un ou plusieurs mécanismes configurés pour détecter la présence du dispositif médical à l'intérieur de la cavité ;
**caractérisé en ce que**
l'un ou plusieurs mécanismes incluent un système de détection infrarouge (130) présentant un ou plusieurs émetteurs infrarouges (132) configurés pour générer un faisceau infrarouge à travers la cavité et un ou plusieurs capteurs infrarouges (134) configurés pour détecter le faisceau infrarouge, les émetteurs infrarouges et les capteurs infrarouges étant en communication avec une console (140).

2. Système de magnétiseur selon la revendication 1, dans lequel la signature magnétique est unique.

3. Système de magnétiseur selon la revendication 1 ou la revendication 2, dans lequel l'un ou plusieurs mécanismes incluent un commutateur mécanique (160) configuré pour passer entre une configuration non magnétisée et une configuration magnétisée.

4. Système de magnétiseur selon l'une quelconque des revendications 1 à 3, dans lequel la signature magnétique inclut une signature magnétique monopolaire et/ou le magnétiseur inclut un lecteur RFID (170) couplé au corps de magnétiseur ou contenu à l'intérieur de la cavité, le lecteur RFID étant en communication avec la console ; et facultativement dans lequel le dispositif médical inclut une étiquette RFID (172) configurée pour être détectée par le lecteur RFID.

5. Système de magnétiseur selon l'une quelconque des revendications 3 ou 4, dans lequel le commutateur mécanique inclut une charnière (162) couplée à chacun du commutateur mécanique et d'un bras de levier (164), le bras de levier faisant saillie dans la cavité et étant configuré pour être contacté par le dispositif médical pour faire passer le commutateur mécanique de la configuration non magnétisée à la configuration magnétisée ; et facultativement
dans lequel la configuration magnétisée inclut le commutateur mécanique déplacé à distance de l'évidement et du corps de magnétiseur ; ou dans lequel le commutateur mécanique se trouve à l'intérieur d'un évidement à l'intérieur du corps de magnétiseur ; et en outre facultativement dans lequel la configuration non magnétisée inclut le commutateur mécanique est perpendiculaire au bras de levier et se trouvant entièrement à l'intérieur de l'évidement.

6. Système de magnétiseur selon l'une quelconque des revendications 1 à 5, dans lequel le dispositif médical incluant des éléments ferreux est choisi dans le groupe constitué d'une aiguille, d'un cathéter et de combinaisons de ceux-ci.

7. Système de magnétiseur selon l'une quelconque des revendications 2 à 6, dans lequel le magnétiseur inclut un ou plusieurs capteurs en communication avec la console, l'un ou plusieurs capteurs étant configurés pour détecter et suivre le dispositif médical en utilisant une détection inductive ou une détection optique.

8. Système de magnétiseur selon l'une quelconque des revendications 2 à 7, dans lequel la console inclut un ou plusieurs processeurs (142), une source d'énergie (144), un support lisible par ordinateur non transitoire (146) et une pluralité de modules logiques ; et facultativement dans lequel l'un ou plusieurs aimants incluent un ou plusieurs électroaimants (128) en communication avec la source d'énergie, ou dans lequel la console est en communication sans fil avec un ou plusieurs dispositifs informatiques ou un système de dossier médical électronique.

9. Système de magnétiseur selon la revendication 8, dans lequel la pluralité de modules logiques, lorsqu'ils sont exécutés par le processeur, sont configurés pour effectuer des opérations incluant :
l'activation de l'un ou plusieurs capteurs infrarouges et de l'un ou plusieurs émetteurs infrarouges ;
la détection de l'étiquette RFID du dispositif médical ;
la détection de l'un ou plusieurs faisceaux infrarouges provenant de l'un ou plusieurs émetteurs infrarouges ;
l'activation de l'un ou plusieurs capteurs ; et
la détection du dispositif médical en utilisant l'un ou plusieurs capteurs par détection inductive ou détection optique.

10. Procédé destiné à imprimer une signature magnétique sur un dispositif médical (110) présentant des éléments ferreux en utilisant un système de magnétiseur (100), comprenant :
la détection du dispositif médical (110) avec un système de magnétiseur (100) présentant un ou plusieurs mécanismes configurés pour détecter la présence du dispositif médical dans lequel l'un ou plusieurs mécanismes incluent un système de détection infrarouge (130) présentant un ou plusieurs émetteurs infrarouges (132) configurés pour générer un faisceau infrarouge à travers une cavité du magnétiseur et un ou plusieurs capteurs infrarouges (134) configurés pour détecter le faisceau infrarouge, les émetteurs infrarouges et les capteurs infrarouges étant en communication avec une console (140) ;
le fait de placer le dispositif médical à l'intérieur de la cavité (126) du magnétiseur ;
le fait de confirmer que le dispositif médical est à l'intérieur de la cavité ; et
le fait d'imprimer la signature magnétique sur le dispositif médical.

11. Procédé selon la revendication 10, dans lequel la signature magnétique est unique et/ou
dans lequel le système de magnétiseur inclut un magnétiseur (120) présentant un corps de magnétiseur (122) définissant la cavité (126), le corps de magnétiseur présentant une ouverture de corps (124) en communication avec la cavité et un ou plusieurs aimants configurés pour imprimer la signature magnétique sur le dispositif médical à l'intérieur de la cavité ;
le magnétiseur présentant un ou plusieurs capteurs (180) en communication avec une console (140), l'un ou plusieurs capteurs étant configurés pour détecter et suivre le dispositif médical en utilisant une détection inductive ou une détection optique ;
et un commutateur mécanique (160) configuré pour passer entre une configuration non magnétisée et une configuration magnétisée ; et facultativement
dans lequel le magnétiseur inclut un lecteur RFID (170) couplé au corps de magnétiseur ou contenu à l'intérieur de la cavité, le lecteur RFID étant en communication avec la console et configuré pour détecter une étiquette RFID (172) couplée au dispositif médical ; et
en outre facultativement dans lequel la détection du dispositif médical inclut l'utilisation du lecteur RFID pour détecter l'étiquette RFID du dispositif médical.

12. Procédé selon l'une quelconque des revendications 10 ou 11, dans lequel le placement du dispositif médical à l'intérieur de la cavité du magnétiseur inclut le fait d'insérer de manière coulissante le dispositif médical à l'intérieur de la cavité.

13. Procédé selon l'une quelconque des revendications 11 ou 12, dans lequel le système de détection infrarouge confirme que le dispositif médical est à l'intérieur de la cavité lorsque le dispositif médical rompt le faisceau infrarouge à travers la cavité.

14. Procédé selon l'une quelconque des revendications 11 à 13, dans lequel la confirmation que le dispositif médical est à l'intérieur de la cavité inclut le fait de confirmer par un commutateur mécanique couplé au magnétiseur, passé d'une configuration non magnétisée à une configuration magnétisée ; et facultativement, dans lequel le commutateur mécanique se trouve à l'intérieur d'un évidement du corps de magnétiseur, présentant une charnière (162) couplée au commutateur mécanique et à un bras de levier (164), le bras de levier faisant saillie dans la cavité et étant configuré pour être contacté par le dispositif médical.

15. Procédé selon l'une quelconque des revendications 10 à 14, dans lequel l'impression de la signature magnétique sur le dispositif médical inclut le fait d'imprimer la signature magnétique sur le dispositif médical lorsque le dispositif médical est retiré de la cavité ; et facultativement dans lequel la signature magnétique inclut une signature magnétique monopolaire.
